# EUROPEAN PATENT APPLICATION

(11) **EP 1 367 508 A1**
(43) Date of publication of application: **03.12.2003**
(21) Application number: 03252902.6
(22) Date of filing: 09.05.2003
(51) Int. Cl.: G06F 17/30, G06F 19/00

(54) **System and methods for extracting pre-existing data from multiple formats**

(30) Priority: 22.05.2002 US 154524
(71) Applicant: Agilent Technologies, Inc., Palo Alto, CA 94306 (US)
(72) Inventor: Vailaya, Aditya, No.109 Santa Clara, CA 95054 (US); Adler, Annette Marie, Palo Alto, CA 94301 (US); Kuchinsky, Allan, San Francisco, CA 94127 (US)
(74) Representative: Tollett, Ian

(57) **Abstract**

A system, tools, software and methods for importing data from multiple sources and of multiple formats and categories, extracting (S3, S4) relevant data from the same, and representing the relevant data in a local format (S5) that can be used for direct comparisons of data across diverse data types or categories and for overlaying (S7) one or more data types over another. Reverse mapping (S9) of relevant data into a different data type or category can also be performed.

## Description

The present invention pertains to a method of facilitating direct comparisons between disparate data formats and categories, and to software systems supporting data gathering and interpretation, and particularly those used in comparing disparate or diverse types or categories of data.

The advent of new experimental technologies that support molecular biology research have resulted in an explosion of data and a rapidly increasing diversity of biological measurement data types. Examples of such biological measurement types include gene expression from DNA microarray or Taqman experiments, protein identification from mass spectrometry or gel electrophoresis, cell localization information from flow cytometry, phenotype information from clinical data or knockout experiments, genotype information from association studies and DNA microarray experiments, etc. This data is rapidly changing. New technologies frequently generate new types of data.

High-throughput techniques are generating huge amounts of biological data which are readily available, but which must still be interpreted. Experiments that measure thousands of genes and proteins (microarray, imminent protein-array technologies, etc.) simultaneously and under different conditions are becoming the norm in both academia and pharmaceutical/biotech companies. A large number of these experiments are conducted in an attempt to solve a piece of the puzzle, that of understanding biological processes. Biologists are in need of tools that help them establish relationships between these heterogeneous data, and extract, build and verify interpretations and hypotheses about these data.

In addition to data from their own experiments, biologists also utilize a rich body of available information from internet-based sources, e.g. genomic and proteomic databases, and from the scientific literature. The structure and content of these sources is also rapidly evolving. The software tools used by molecular biologists need to gracefully accommodate new and rapidly changing data types.

A number of literature (Pubmed, USPTO patent database) and pathway databases (Bind, EMP, KEGG, TransFac, TransPath etc.) have been developed (both public domain and proprietary) that allow users to query and download scientific articles and biological models of interest However, these abstracts/publications or the biological models that are returned for a specific query are static files that do not necessarily link to other data (either in house or publicly available). In other words, since these are centrally maintained (e.g., EBI, NCBI, etc.), they are static files and do not allow arbitrary and dynamic overlay of multiple data types on their content. Specifically, none of these databases allow overlay of proprietary data (experimental or other kind) on the returned query results. A major limitation of these databases is a lack of standard representation of their contents, and hence their content is not easily machine interpretable. Therefore, relating information imported from these existing databases to experimental data-and interpretations is extremely cumbersome.

Although some tools have been developed for overlaying a specific type of data onto a viewer, they are very limited in their approach and do not facilitate the incorporation of diverse data types whatsoever. For example, a tool called EcoCyc [http://ecocyc.org]. is capable of overlaying gene expression data on pathways, but is limited to only gene expression data. Another example known as GeneSpring, by Silicon Genetics [http://www.sigenetics.com], is available for overlaying gene expression data on genomic maps, but again, is limited to this specific application.

Because of the vast scale and variety of sources and formats of these various types of data, an enormous number of variables must be compared and tested to formulate and validate hypotheses. Thus, there is a need for new and better tools that facilitate the comparisons of these data in formulating and validating/invalidating hypotheses.

Currently, there do not exist any systems that automatically or semi-automatically link existing scientific text and biological models (the legacy data) to other types of data (both proprietary and public). Publications, patents and other forms of scientific text, along with biological models are great repositories of information related to the current understanding of the functioning of biological processes. With the high-throughput experiments and their results that scientists have to deal with, there is a need to identify information about entities of interest from the existing vast literature and available/known biological models, and be able to verify/validate these using proprietary experimental results, or design the next set of experiments.

A method of facilitating direct comparisons between disparate data formats and categories is provided to include extracting relevant data from a first data set having a first data format and characterized in a first data category; converting the relevant data from the first data set to a local format, extracting relevant data from a second data set; converting the relevant data from the second data set to the local format; and
comparing the relevant data from the first data set with the relevant data from the second data set using the local format. One implementation of the local format may be a standardized/reduced grammar.

The second data set may have a second data format which is different from the first data format, a second data category which is different from the first data category, or both.

Further, the relevant data in the local format from the first data set is linked with relevant data that matches it in the locally formatted relevant data from the second data set.

The present invention also provides various methods of overlaying data from one or more data sets onto data from another data set, or vice versa. The overlay may be visualized to compare the data from the disparate sources. Further, a visual indicator may be provided on the data upon which the overlay is produced, to further facilitate the comparison of data.

The actual data from one data set may be overlaid on the actual data of another data set, based on the local formatting and linking of the same, to enable a literal comparison thereof.

In particular, categories of scientific text data, experimental data and biological models may be processed by the system, methods and tools according to the present invention.

Extraction of relevant data from the various categories may be performed automatically, semi-automatically or manually, for inputting the relevant data to a local format module for representation of the relevant data in the local format.

The local format may take the form of a programming language, grammar or Boolean logic, for example.

Additionally, the locally formatted relevant data may be used for reverse-mapping the locally formatted relevant data to construct the relevant data into a data set characterized according to a different category than the category characterizing the data set from which the relevant data was originally extracted.

A system for visualizing biological relationships from data selected among diverse data types is provided to include means for accessing data sets having diverse data types; means for extracting relevant data from each data set; respectively; and means for converting the relevant data to a local format.

The system may further include means for linking the relevant data in the local format from each data set with relevant data that matches it in the locally formatted relevant data from the other data sets, as well as means for overlaying the relevant data from one or more of the data sets onto another of the data sets, based on the local formatting and linking.

Still further, the system may include means for automatically comparing the overlaid relevant data with the relevant data upon which it is overlaid.

Means for alerting a user may be provided to alert the user when the means for automatically comparing determines that there is a discrepancy found by the comparison.

Means for reverse-mapping locally formatted relevant data from a first of diverse data types to a data set having a second of the diverse data types may also be provided.

A computer readable medium carrying one or more sequences of instructions from a user of a computer system for visualizing biological relationships from data selected among diverse data types is provided, wherein the execution of the one or more sequences of instructions by one or more processors cause the one or more processors to perform the steps of accessing data sets having diverse data types; extracting relevant data from each data set; respectively; and converting the relevant data to a local format.

Further, the medium may be executed to perform the step of linking the relevant data in the local format from each data set with relevant data that matches it in the locally formatted relevant data from the other data sets.

Still further, overlaying the relevant data from one or more of the data sets onto another of the data sets, based on the local formatting and linking may be performed.

Automatic comparison of the overlaid relevant data with the relevant data upon which it is overlaid may also be provided. Additionally, the user may be alerted when the means for automatically comparing determines that there is a discrepancy found by the comparison.

Execution of the medium may also be carried out to perform reverse-mapping of locally formatted relevant data from a first of the diverse data types to a data set having a second of the diverse data types.

Among other advantages, the present invention allows users to automatically overlay information on scientific text, biological models and experimental data, including imported versions of each of these categories that already have a fixed format.

The present invention allows users to automatically overlay information on imported biological models, scientific text, or experimental data, and to visualize information relevant to entities (e.g., molecules/genes/proteins) of interest within scientific text, biological models or experimental data while browsing the same.

Thus, the present invention allows users to visualize comparisons between interpretations from experimental data, textual data and biological models.

Mapping onto a standardized/reduced grammar can aid the user in transforming data/information of one kind to another, such as transforming text into pathway diagrams and vice versa.

Overlays can also aid in building interpretations of the biological process, generating new hypotheses, and designing further experiments.

These and other objects, advantages, and features of the invention will become apparent to those persons skilled in the art upon reading the details of a number of preferred embodiments of the invention as more fully described below.

Fig. 1 is an architectural schematic of a system according to the present invention.

Fig. 2 is a flow chart showing data mining of scientific text data, conversion of the mined data to the local format, and use of the local format to overlay and reverse-map.

Fig. 3 is a flowchart showing typical flow paths taken by the system in constructing data in a local format representation, and in using the local format representation to perform overlays and/or reverse mapping.

Before the present system, tools and methods are described, it is to be understood that this invention is not limited to particular data sets, commands or steps described, as such may, of course, vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It must be noted that as used herein and in the appended claims, the singular forms "a", "and", and "the" include plural referents unless the context clearly dictates otherwise. Thus, for example, reference to "a step" includes a plurality of such steps and reference to "the pathway " includes reference to one or more pathways and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

Interpretations/hypotheses which are developed in story or textual form or diagrammatic form may be dependent upon many different cellular processes, genes, and various expressions of genes with resultant variations in protein abundance. Correlation and testing of data against these hypotheses is becoming increasingly more tedious and lengthy with the increased automation of the ways in which gene and other data is generated (e.g., microarrays, mass spectroscopy. etc.). The present invention provides a system, tools and methods for standardizing heterogeneous forms of information, so that they may then be compared and visualized to validate/invalidate data and hypotheses, as well as develop new hypotheses/refme exist hypotheses.

It is also very useful to correlate experimental data with other representations of biological data, for example correlating gene expression data with genes on a chromosome map view, or with proteins in a pathway diagram. Still further, many biologists work with textual descriptions of hypotheses and interpretations of data; and would be useful to correlate experimental data with these textual representations, as well as graphical representations. In cases where textual and diagrammatic representations are built in conjunction with or after the collection of experimental data, making correlations between the textual and diagrammatic representations with the experimental data can be accomplished using generalized data overlays, as disclosed in co-pending, commonly owned Application (Attorney ref. N13209) claiming priority from US 10/155,616 filed concurrently herewith and titled "System and Methods for Visualizing Diverse Biological Relationships", the entirety of which is incorporated herein by reference thereto. However, if the textual and diagrammatic representations are pre-existing, establishing relationships between these pre-existing representations and experimental data or other data in another format cannot be automatically accomplished using existing tools because of the incompatibility between data types that is almost always existent.

The present invention provides a system, methods and tools to import pre-existing data (scientific text or biological models) and to extract and represent the imported content in a common format for facilitating visualization of these relations via overlays, where data from one source or view is superimposed upon data items in a different view. Other examples of these kinds of overlays could include analytical plots, such as overlaying color coding or symbols onto log ratio plots. This would permit visualizing clinical data on a typical gene expression graph and similar sorts of visualizations.

The present invention provides a system that allows users to import scientific text and existing biological models, and facilitates overlay of experimental data or other information over these. The system extracts semantic information and represents it in a restricted grammar/language, referred to as the local format. The local format can also link information from all three categories, and may be carried out automatically. The information that results in the local format can then be used as a precursor for application tools provided to compare the experimental data with existing textual data and biological models, as well as with any textual data or biological models that the user may supply. Further, experimental data can be imported into the system and compared in the same manner. Applications for visualizing the comparisons may be employed which access the information from the local format, and which can then automatically perform overlays, and other functions, for example.

Biologists and researchers access various types of information which the present invention approaches by grouping these various sorts in three general categories. The first is referred to as "scientific text", which includes stories, scientific reports, abstracts, journals, patents, scientific publications (on web) or other web pages. The second category is referred to as "biological models", which includes diagrammatic representations of biological information, metabolic pathways (enzymes which control reactions), signal transduction pathways (graphically describing how a signal moves to the level where cell recognizes it to produce a particular protein), gene networks, transcription factors (promoter regions, gene is transcribed to produce a protein); proteins that bind to DNA to lead to the transcription, protein-protein interactions (proteins that can bind to one another, don't define any particular factor, but there might be some significance to this binding), interactions between molecules, compounds, or drugs (diagrammatic/graphical representations of how a compound interacts with a gene, protein or other compounds, etc.), and the like. The third main category is referred to as "experimental data", which may include genomic data (e.g., microarrays, Taqman data), proteomic data (e.g., mass spectrometry data, gel data, protein arrays), or any other data that a researcher desires to correlate with the literature and which can be converted to tabular data, such as clinical data or the like.

The present invention converts the three categories of information (heterogeneous data) to a format, which is referred to as the "local format" so that they can be interchanged, correlated and overlaid with one another. The local format used may be a computing language, grammar or Boolean representation of the information which can capture the ways in which the information in the three categories are represented.

The information that has been converted to local format can then be used to make direct comparisons between experimental data, biological models and scientific text and visualization tools may be used to relate any combination or all of these multiple pieces (such as text, diagram, experimental data, interpretation, etc.) together into a single view providing insights to solve the puzzle posed by a hypothesis.

The heterogeneous data may include information about "entities" (e.g., sequences/genes/proteins/molecules and information about protein-protein interactions, post-translational modifications of proteins, expression of genes, presence of proteins in cells/tissues, protein localization, biological pathways and other diagrams of biological processes, wherein the "relationships" between entities may or may not be explicitly stated. For example, textual data (e.g., scientific publications, reports, etc.) may explicitly state that "gene A upregulates gene B", or alternatively, there may be a textual description which could be interpreted by the researcher to conclude that gene A upregulates gene B. In the case of a pathway, it may likely explicitly show (in some format) that gene A upregulates gene B. Experimental data may explicitly show the relationship or may be interpreted to conclude that gene A upregulates gene B. In order to make sense of all these data, it is important to relate them, such as by extracting the entities and relationships and converting them to a local format.

The ability to relate (or provide tools to facilitate the relation) scientific text and existing biological models to experimental data allows the user to develop valuable interpretations of the biological processes. The nature of this relationship of published results or biological models to experimental data can reveal ideas for further experiments, setting up proper controls for experiments, etc.

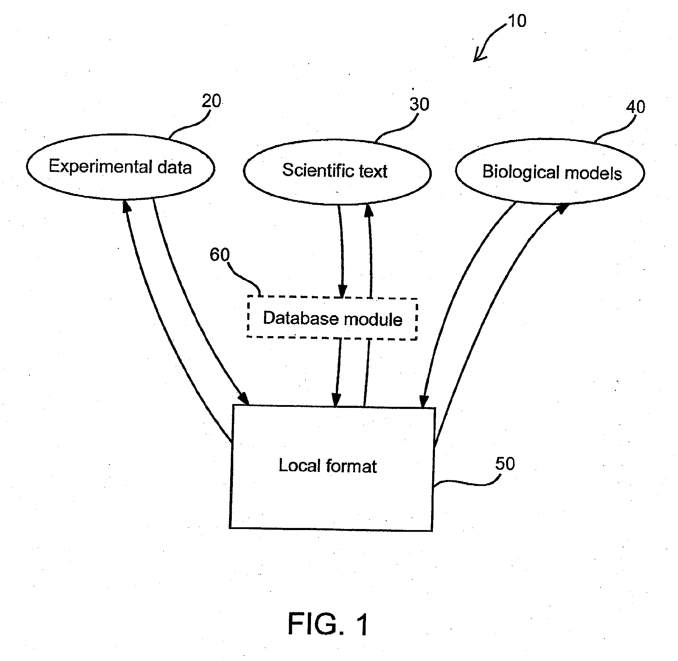

Referring to Fig. 1, an architectural schematic of a system 10 according to the present invention is shown. A first tool or application 20 is provided for storing experimental data which may be either downloaded from an external source, or loaded from a local database of the user or other local database. An example of a tool used for this purpose would be a results viewer of the type described in co-pending Application based on US 10,155,616
and titled "System and Methods for Visualizing Diverse Biological Relationships", and in co-pending , commonly owned Application (Application number not yet assigned, Attorney Docket No. 10020613-1) filed concurrently herewith and titled "Database Model, Tools and Methods for Organizing Information Across External Information Objects", the entirety of which is incorporated herein by reference thereto. A tool or application 30 is provided for downloading scientific text from the world wide web, ftp or for accepting scientific text from any other digitally provided source. An example of a tool 30 is a text viewer or text editor. A story editor may also be used in conjunction with or in place of such an editor, such as the story editor described in commonly-owned, co-pending Application Serial No. 09/863,115, filed May 22, 2001 and titled "Software System for Biological Storytelling", which is incorporated herein by reference thereto, in its entirety, and in co-pending, commonly owned Application (Application number not yet assigned, Attorney Docket No. 10020613-1) filed concurrently herewith and titled "Database Model, Tools and Methods for Organizing Information Across External Information Objects". Further, a tool or application 40 is provided for downloading biological models from the world wide web, ftp or from any other digitally provided source. An example of a tool 40 is a diagram editor as described in co-pending Application based on US 10/155,616
and titled "System and Methods for Visualizing Diverse Biological Relationships" and in co-pending Application (Application number not yet assigned, Attorney Docket No. 10020613-1) filed concurrently herewith and titled "Database Model, Tools and Methods for Organizing Information Across External Information Objects". Co-pending, commonly owned Application (Application number not yet assigned, Attorney Docket No. 10020150-1) filed concurrently herewith and titled "System and Methods for Extracting Semantics from Images" also relates to downloading of biological models and is incorporated herein, in its entirety, by reference thereto.

In addition to downloading and storing the various categories of data described above, tools 20, 30 and 40 may also provide the capability to the user to manually construct data according to the respective category types. For example, using tool 40, the user can manually put together a pathway diagram, using external data, originally created input from the user, or data stored in any of the other applications in the system such as tools 20 and 30, or from the local format module 50 described below. Tools 20 and 30 can be used similarly.

Upon loading any or all of the tools 20, 30 and 40 with data from a corresponding category, the local format module 50 extracts relevant information from each of the categories, imports the relevant information and converts the information to the local format. The standardized representation provided by the local format allows for easy extraction of relationships from multiple data formats (scientific text, biological models, experimental results, etc.). These relationships can be visualized via overlays of experimental data onto scientific text and biological models, and vice versa. For example, data from the experimental data tool 20 having been extracted and converted to the local format can be automatically accessed from the local format module 50 by the scientific text application 30, where entities from the experimental data matching entities in the scientific text can be overlaid on the scientific text in the scientific text application and viewed by the user. The actual overlay viewed by the user may be in the form of an icon, for example. Different icons may be overlaid on the textual data to give different meanings. For example, a first icon could mean that supporting data is found in the experimental data and a second icon could mean that contradictory data is found in the experimental data. Alternatively, or additionally, the icons could be colored differently, for example. Clicking on one of these icons, when present in the text viewer, would take the view to the exact location in the experimental data where the supporting or contradictory data is located. The exact location is facilitated by going back through the local format module and the links between the textual data and experimental data point to the exact location in the experimental data. Alternatively, or additionally, a hyperlink can be inserted into the text linking that portion of the scientific text to the exact location or locations in the experimental data that corresponds to the text.

Relationships associated with those entities can be compared between the experimental data and the scientific text to help in either supporting or opposing a hypothesis concerning such relationship. "

Not only does the local format module 50 convert the relevant data to the local format, but it also links matching entities from different categories, as well as relationships associated with those entities. In this way, the overlaying or other comparison process becomes automatic after establishing each category of data in the local format and linking the matches. Further, the system can then "build on itself' by building additional links every time new data is accessed (scientific text, biological models or experimental data) to the data already existing and linked in the local format. In this way, the system provides links to other public/proprietary data regarding entities (molecules, genes, proteins, etc.) of interest.

Similarly, data from the experimental data tool 20 having been extracted and converted to the local format and linked to data converted to the local format from a biological model in the biological models application 40 can be automatically accessed from the local format module 50 by the biological models application 30, where entities from the experimental data matching entities in the biological model can be overlaid on the scientific text in the scientific text application and viewed by the user. Again, icons or hyperlinks or some other visual indicator can be overlaid on the scientific text, which the user can select by mouse clicking or other selection method to access the exact location of the biological model that corresponds to the text. Relationships associated with those entities can be compared between the experimental data and the biological model to help in either supporting or opposing a hypotheses concerning such relationship.

In a like manner, data from the scientific text having been extracted and converted to the local format and linked to data converted to the local format from a biological model in the biological models application 40 can be automatically accessed from the local format module 50 by the biological models application 40, where entities from the scientific text matching entities in the biological model can be overlaid on the biological model in the biological models application 40 and viewed by the user. One visual way of indicating the overlay in this instance is to highlight or change the color of that portion of the biological model to which the relevant text relates. By clicking on or otherwise selecting the highlighted or colored portion of the model, the user will then pull up a view of the exact portion of the text that contains the relevant information. Additionally, when color coding is used, a first color can be used for supporting text and a second color can be used for text that refutes or opposes the portion of the hypotheses highlighted in the biological model. Relationships associated with those entities can be compared between the scientific text and the biological model to help in either supporting or opposing a hypothesis concerning such relationship.

Overlaying or other visual comparisons of the data from the scientific text on the biological model can likewise be automatically performed after that. Also, overlays of either or both of the data from the scientific text application 30 and biological model 40, after being converted to the local format and linked to the local format of the experimental data, can be accessed by the experimental data tool 20 through the local format module 50 and performed on the experimental data. Again, icons, hyperlinks and/or coloring can be used to overlay the experimental data which can be selected to pull up a view of the appropriate text or model data that corresponds to that portion of the experimental data. Of course, different overlay symbols may be used for each of the biological model data, textual data (and the experimental data, when it is being overlaid on one of the other categories). Both biological model data and experimental data can be overlaid or compared on the scientific text simultaneously, after appropriate conversion of the relevant data from each category into the local format and linking matching data/entities. Likewise, both experimental data and scientific text can be overlaid or compared on the biological model simultaneously, after appropriate conversion of the relevant data from each category into the local format and linking matching data/entities.

Although the applications 20, 30 and 40 have been described as carrying out the downloading and storing functions of the respective categories of data, as well as performing the comparisons between data, such as overlaying and visualizing, it is noted here that although such an architecture is compact and convenient, it is not required by the invention. For example, visualization/overlay tools or applications that are separate from the tools 20,30,40 for downloading and storing the data may be used.

In the case of textual data, although scientific text tool 30 may be a story editor as noted above, into which case a story could be loaded or manually constructed, and from which relevant information could be directly extracted to represent in the local format, when the textual data downloaded is a scientific text article or some other textual document input or downloaded from the world wide web, for example, a data mining module, such as that described in Application Serial No. 10/033,823 may be employed to search for relevant entities and relationships and extract them from the textual data, which are then supplied to the local format module for representation of the information in the local format. Application Serial No. 10/033,823, filed on December 19, 2001 and titled "Domain Specific Knowledge-Based Metasearch System and Methods of Using" is incorporated herein in its entirety, by reference thereto. Using this type of data mining module may require some manual intervention to convert the mined information to the local format. For example, this type of data mining may extract a portion of a paragraph containing one or more entities (noun(s)) of interest. The user would then read the extracted excerpt and make an interpretation as to any relationship or relationships (verb(s)) between entities, or effect on one entity that may be described in the excerpt, and either extract such relationship for the text, or manually enter the relationship to be described in the local format.

Alternatively, data mining module 60 may employ natural language processing (NLP) techniques for a more automated conversion of the textual data to the local format, where the NLP data mining module is supplied with a database of relevant nouns and verbs and can make limited interpretations of the text to extract relevant nouns, verbs and pre-defined templates of sentences, and can make limited interpretations of the text to extract relevant nouns and verbs automatically, and feed them to the local format module 50 where they are represented in the local format.

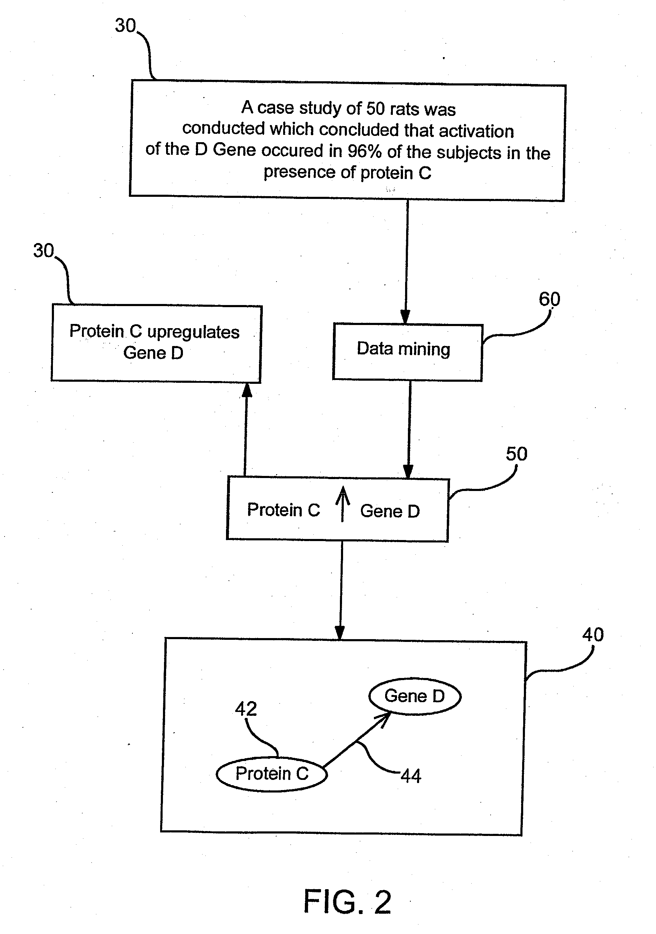

The standardized representation provided by the local format also allows for easy transformations of data of one kind to another. For example, a list of scientific text can be transformed or reverse-mapped to a pathway diagram or other graphical biological model and vice versa. For example, referring to Fig. 2, a scientific article provides text in the scientific text module 30 that can be interpreted to determine that protein C activates gene D. Using a data mining module, the nouns "protein C" and "gene D" are extracted. If using an NLP module, the verb "activating" or "upregulating" may also be automatically extracted. Otherwise, the user may view the portion of the text from which the nouns were selected upon being guided there by the data mining module, read the relevant portion of the text, and conclude that the verb "activates" or "upregulates" should be manually input to describe the relationship between protein C and gene D. As another alternative, the user may read the article from the text viewer 30 and manually select all of the nouns protein C, gene D and the verb "activates". By any of these techniques, the nouns and verb are then supplied to or accessed by the local format module 50 which represents the relevant data in the local formats The local format shown in Fig. 2 uses an "up arrow" to indicate the relation meaning "activates or upregulates, but the invention is not limited to this nomenclature, as noted above. One alternative would be to use the word "upregulates" to signify an upregulation or activation. Whatever the nomenclature, the local format simplifies the information to put it into a format that can be used in all three categories of information. Thus, for example, "activation" and "upregulation" would likely be equated in the local format to be represented by the same symbol.

The local format may next be accessed by a diagram editor 40 which may automatically construct a pathway diagram using the information provided by the local format module 50. Symbolic representations 42 of the nouns may use the same depiction with labeling, as shown, or, alternatively, may use differently shaped symbols to indicate a protein versus a gene. Further alternatively, or additionally, the depictions may have differential coloring. The verb is represented in this case by the arrow 42, with the arrow head pointing toward gene D to indicate that the protein C is acting to effect the gene D. Additionally, the arrow may be colored to indicate the action, such as it may be colored red to indicate upregulation, for example, whereas a green arrow, for example might indicate downregulation, and a black arrow might indicate neutrality or no activity. Alternatively, the user can access the local format information, using the diagram editor and select the nouns and verbs to manually construct a pathway diagram as shown.

The process shown in Fig. 2 can make use of the local format information to build any of the three categories of data types. Thus, for example, after constructing the local format language form the text article as described above, the local format language could be accessed by a story editor 30, for example, and the information is then converted to story grammar in the story editor 30 as shown in Fig. 2. Similarly, the local format language could be used to construct a column or row in a data table in a results viewer 20.

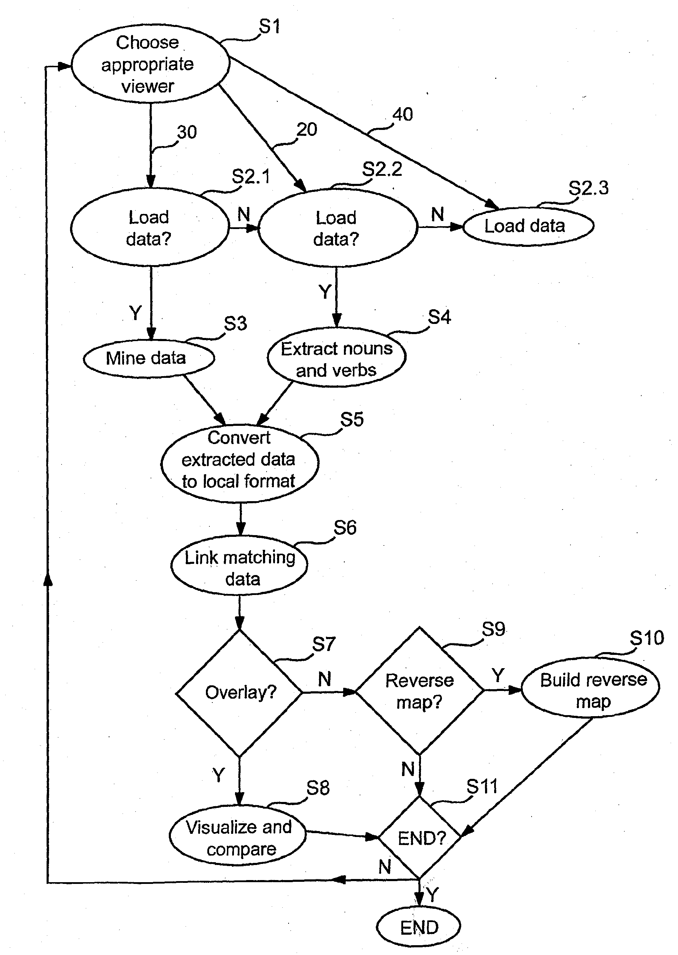

Fig. 3 is a flowchart showing typical flow paths taken by the system in constructing data in a local format representation, and in using the local format representation to perform overlays and/or reverse mapping. In step S1, the appropriate viewer is selected for viewing and processing the type of data that the user is interested in accessing. As described above, if scientific text is to be accessed, a tool 30 such as a text editor, text viewer of story editor is selected. If experimental data is to be downloaded, viewer 20 is selected, and if pathway or other graphical data is to be downloaded, a viewer 40 of the type described above is selected. In step S2 (steps S2.1, S2.2, S2.3), the data is loaded into the viewer that has been selected. The loading step may be accomplished in a variety of ways including downloading information from a source available from the world wide web or ftp, loading from an internal source such as a diskette, hard drive or other storage medium, or the data may be manually input by the user, for example. After loading the data into the appropriate viewer, if viewer 20 was selected the data is scientific text. In the case where a viewer 30 was selected and the data downloaded is scientific text, the process moves to step S3, where a data mining tool, as described above is used to mine relevant nouns and possibly relationships between these nouns. For example, in reading a scientific text document that says gene A upregulates gene B, the data mining tool can extract the nouns "gene A" and "gene B", as well as the verb or relationship "upregulates". For more complex language that must be interpreted to include that gene A upregulates gene B, this may still possibly be automatically accomplished using an NLP tool. In the worst case, using any of the data mining tools, the nouns "gene A" and "gene B" can still be automatically extracted. After that the user can access those portions of the text that contain the extracted nouns, read those portions and make interpretations as to the relationships being described. After that, the user manually inputs the verbs or relationships.

If the viewer selected was a viewer 20 and the data loaded is experimental data, then.-the process moves to step S4 where the relevant information is extracted using an experimental data tool 20. Some forms of experimental data lend themselves to automatic extraction of both nouns and verbs. For example, nouns and verbs can be automatically extracted from gene expression experimental data. The nouns are clearly present, and correlation can also be determined from the data, (e.g., when gene A goes up, then gene B goes up). State information is also readily automatically extracted. Again, in the worst case, where relationships or verbs cannot be automatically extracted from the experimental data, the nouns generally can be automatically extracted, and then the user can access the experimental data, interpret it, and supply the appropriate verbs or relationships between the extracted nouns to the local format module 50.

If a viewer 40 was selected at step S1, and pathway or other biological model or graphical data was loaded at step S2.3, the process moves to step S4 where relevant data (e.g., nouns, verbs) are extracted from the biological model, either automatically, semi-automatically (e.g., as in the case where nouns are extracted automatically and the user interprets the biological model and manually interprets the results of the interpretation, which may include inputting verbs and possibly additional noun) or manually. It should be noted here that relevant data extraction and input to the local format module may be optionally performed entirely manually by a user for any of the categories, by accessing the appropriate viewer containing the loaded data, interpreting the data, and manually inputting relevant data resultant from the interpretation.

After completion of any of steps S2.1 and S3; S2.2 and S4; or S2.3 and S4, the relevant data is input to the local format module 50 which converts the relevant data to the local format at step S5. As noted above, the local format is a much reduced expression of what may appeared in the scientific text, for example. The local format, however, puts the data from each of the data categories on a "level playing field", so that direct comparisons may be made between the data from each of the categories.

The local format module further links the converted data with any other information the system has on data which was converted at step S6. In this example, gene A and gene B and their interrelationships are linked with any other existing data in the system that pertains to gene A, gene B, or any interrelationship between the two.

At step S7, the user is given the opportunity to perform data overlays, if desired. In this way, the user is afforded the capability of overlaying one or more data types (categories) over an existing category of data. For example, the user can overlay experimental data over a full scientific textual document in a text viewer using this function. In its simplest form, the overlay would provide a hyperlink in the appropriate parts of the text that relate to experimental data of the user. This is made possible by the links already existing in the local format between the matching data supplied by that scientific article and the experimental data. By selecting or clicking on the hyperlink, the user is then taken directly to the data that relates to the scientific text where the hyperlink (or other icon, or visual display, as described above) is located. Next, the actual data from the experimental data can optionally be overlaid on the scientific text (step S7), for a direct visual comparison of the two data categories (step S8).

Thus, overlaying, whether only by providing a hyperlink or icon and accessing the linked data for side to side comparison, or by directly overlaying the actual data from one category over another, allows visualization (step S8) by the user to see whether the two sets of data support one another, or whether there is opposition or conflict between the two categories of data. For example, if the scientific text document says that gene A upregulates gene B, but the user's experimental data says that it doesn't, the system may display a visual indicator to alert the user to this discrepancy. For example, the system can blink the text in the scientific text document that is disputed, or display some other indicator (such as color the text red if there is a discrepancy, or color it green if there is an agreement, for example) to show that there is a discrepancy or support. This can all be done automatically once both data categories have been represented in the local format. When a discrepancy is identified, either automatically or manually (by the user viewing and comparing the data), this can be valuable information in helping the user to pinpoint the source of the discrepancy, either by repeating the experiments to generate more experimental data to test the original experimental data and see if the results are repeated, questioning the scientific textual data and having it changed if it is in error, or formulating new hypotheses to account for the discrepancy.

The standardized representation of all the data in the system allows automatic transformation of scientific article to a biological model or experimental data; biological model to experimental data or scientific text or experimental data to scientific text or biological model, as well as reversing these transformations. Thus, if the user does not desire to perform an overlay, at step S9, a reverse mapping procedure can be performed to create another data category using the local format of data from a category existing in the system at S9 and S10. Once visualization and comparison are completed at step S8, or a reverse mapping is completed at step S 110, or neither overlaying (S7) nor reverse mapping (step S9) are selected, the user may wish to exit the process as step S11. Alternatively, the user is given the option to return to step S1, where the same viewer may be used to load an additional data set, or a different type of viewer may be selected to access and load a different type of data to continue processing.

Although the flow chart shows the steps of deciding whether to perform a reverse mapping procedure (step S9) and performing a reverse mapping (step S10) as being performed only after it is decided that an overlay is not desired to be performed (step S7), the system is not limited to this flow path. Thus, for example, the user may have scientific text data in the local format as well as experimental data in the local format, and perform an overlay at step S7. After analysis by overlay and visualization at step S8, the user may then wish to reverse map a pathway diagram (biological model) using one or both of the local formatting for the scientific text document and the experimental data. This is available to the user and the user can perform the reverse mapping after overlaying and visualizing. That is to say, that step S9 is not dependent upon not performing an overlay, but may be performed at any time in the process, as long as there is local formatting for at least one other data category.

While the present invention has been described with reference to the specific embodiments thereof, it should be understood by those skilled in the art that various changes may be made and equivalents may be substituted without departing from the true scope of the invention. In addition, many modifications may be made to adapt a particular situation, data type, network, user need, process, process step or steps, to the objective, and scope of the present invention. All such modifications are intended to be within the scope of the claims appended hereto.

## Claims

1. A method of facilitating direct comparisons between disparate data formats and categories, the method comprising the steps of:
extracting relevant data from a first data set having a first data format and **characterized in** a first data category;
converting the relevant data (S5) from the first data set to a local format;
extracting relevant data from a second data set;
converting the relevant data (S5) from the second data set to the local format; and
comparing the relevant data from the first data set with the relevant data from the second data set using the local format.

2. The method of claim 1, wherein the second data set has at least one of a different data format and a different data category from that of the first data set.

3. The method of claim 1 or 2, further comprising the step of linking (S6) relevant data in the local format from the first data set with relevant data that matches it in the locally formatted relevant data from the second data set.

4. The method of any of claims 1-3, wherein the step of comparing includes overlaying data (S7) from the first data set on data from the second data set, or vice versa.

5. The method of claim 4, wherein the step of comparing further comprises visualizing (S8) an indicator on either the first or second data set produced as a result of the overlaying and comparison with the other of the first and second data sets.

6. The method of claim 4 or 5, further comprising the step of visualizing (S8) the overlaid data from one of the first and second data sets over the other of the first and second data sets to enable a comparison thereof.

7. The method of any of claims 1-6, wherein the second data set is **characterized in** a second data category that is different from the first data category, the method further comprising the step of reverse-mapping (S9) the locally formatted relevant data from the second data set to construct the relevant data into a data set **characterized in** the first data category.

8. The method of any of claims 1-7, wherein the second data set is characterized according to a second data category which is different from the first data category, the method further comprising the steps of:
extracting relevant data from a third data set characterized according to a third data category which is different from the first and second data categories;
converting (S5) the relevant data from the third data set to a local format;
comparing the relevant data from any of the first, second and third data sets with any of the others of the first, second and third data sets, using the local format.

9. The method of claim 8, further comprising the step of comparing two of the first, second and third data sets with a third of the first, second and third data sets simultaneously.

10. A method of facilitating the comparison of disparate data types, the method comprising the steps of:
extracting relevant data from a first data set having been characterized according to a first data category;
converting (S5) the relevant data from the first data set to a local format; and
reverse-mapping (S9) the locally formatted relevant data to construct the relevant data into a second data set characterized according to a second data category which is different from the first data category.

11. A system for visualizing biological relationships from data selected among diverse data types, said system comprising:
means for accessing data sets (20, 30, 40) having diverse data types;
means for extracting relevant data from each data set; respectively; and
means for converting (50) the relevant data to a local format.

12. A computer readable medium carrying one or more sequences of instructions from a user of a computer system for visualizing biological relationships from data selected among diverse data types, wherein the execution of the one or more sequences of instructions by one or more processors cause the one or more processors to perform the steps of:
accessing data sets having diverse data types;
extracting relevant data (S3, S4) from each data set; respectively; and
converting (S5) the relevant data to a local format.
